(19) Europäisches Patentamt
European Patent Office
Office européen des brevets

(11) Publication number: **0 376 077 A2**

(12) **EUROPEAN PATENT APPLICATION**

(21) Application number: 89123145.8

(22) Date of filing: 14.12.89

(51) Int. Cl.5: C07D 209/20, A61K 31/405

(30) Priority: 26.12.88 ES 8803956

(43) Date of publication of application: 04.07.90 Bulletin 90/27

(84) Designated Contracting States: AT BE CH DE FR GB IT LI NL SE

(71) Applicant: LABORATORIOS VINAS S.A.
386, Calle Provenza
E-08025 Barcelona(ES)

(72) Inventor: Buxadé Vinas, Antonio
230, Calle Compte d'Urgell
E-08036 Barcelona(ES)

(74) Representative: Dipl.-Ing. Schwabe, Dr. Dr. Sandmair, Dr. Marx
Stuntzstrasse 16
D-8000 München 80(DE)

(54) **A process for the preparation of a new tryptophane derivative.**

(57) A process for the preparation of a novel tryptophane derivative.

This process, for the preparation of a new tryptophane derivative, is characterised in that L-5-hydrohytryptophane is reacted with zinc oxide or hydroxide, or a corresponding ammonium, alkali metal or alkaline earth metal salt of L-5-hydroxytryptophane is reacted with a zinc salt in a polar solvent.

It is applied in the preparation of compounds having therapeutical activity on the central nervous system.

EP 0 376 077 A2

# A PROCESS FOR THE PREPARATION OF A NEW TRYPTOPHANE DERIVATIVE

## Field of the Invention

The present invention relates to a process for the preparation of a new tryptophane derivative and, more particularly, a L-5-hydroxytryptophane zinc compound.

This new compound is characterised by having the following formula (I):

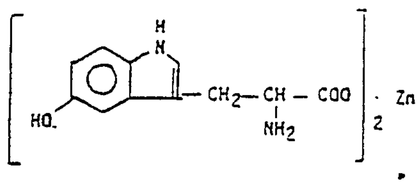

(I)

and is novel as an active substance and, of course, as a therapeutical application.

## Background of the Invention

Tryptophane is known as an essential amino acid having a role in biochemical brain processes. L-5-hydroxytryptophane is a more useful metabolite than tryptophane in clinical applications and also has the advantage of being administered at lower dose levels.

Furthermore, zinc has been reported to be a potentially useful trace element in the treatment of central nervous system disorders as it participates in various enzyme processes.

Nevertheless, the inventor is unaware of any derivative compounds allowing the administration of both components at the same time and in a single dose having been reported.

## Detailed Description of the Invention

The inventor's scientific research has led to the discovery of a new compound of formula (I). Outstanding among the advantages of this new compound is that all its components are non-toxic. A further important point is that the formula (I) derivative is a compound which, because its components are to be found in the human organism, may be considered to be biological or physiological, allowing it to be administered as a dietary treatment, to correct deficits in humans or as a therapeutical treatment to increase the amounts already available in the organism. A further outstanding advantage of this derivative is its capacity to act upon the central nervous system, it being biochemically useful in the synthesis of serotonin, whereby its therapeutical potential is very broad, for example, for the treatment of depressive states, as a modulator of the central nervous system and in other applications .belonging to the psychiatric and neurological medical fields.

The special chemical formula of the new compound according to the invention makes it non-toxic and, in particular, it does not create dependency, a negative feature of other drugs on the market, whereby its prescription as a drug will not require any special psychotropics control.

The process for preparing the new compound of formu1a (I) according to the present invention is characterised in that L-5-hydroxytryptophane (L-5-HTP) of formula (II)

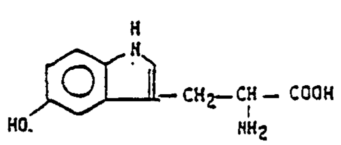

(II)

or an ammonia or alkali metal or alkaline earth metal salt thereof (as such or prepared in situ and unisolated) is reacted with zinc oxide, hydroxide or a zinc salt, respectively, in an equimolar amount or a slight excess of L-5-HPT or of the salt thereof.

To obtain the formula (I) compound of the invention with a high purity and a defined, reproducible composition, the operative method requires certain special precautions. Otherwise, undefined products mixtures occur, due, on the one hand, to the high functionalisation of the formula (II) L-5-HPT, with electron donor groups and acid groups, capable of interacting in both ionic and complexing form with zinc and, on the other hand, to the relative instability of the starting product and even of the zinc derivative in solution or as an isolated solid, particularly in the presence of oxygen.

To avoid these dificulties, it is desirable to prepare the formula (I) compound maintaining an excess of L-5-HPT of formula (II) or of the corresponding salt thereof, during the reaction, which is attained with the addition of the zinc oxide, hydroxide or salt over the solution of L-5-HPT. It is also desirable to work under an inert atmosphere, using short drying times of the end product in the presence of air. The drying is preferably effected under an inert atmosphere or reduced pressure, thereby avoiding colouring of the end product.

The reaction is conducted in polar solvents or mixtures thereof, preferably in water or a mixture of water and a low molecular weight alcohol, preferably methanol.

The temperature may range from room temperature to the reflux temperature of the solvent or mixture of solvents, preferably the reflux temperature.

The zinc reactants may be added in solid form, in portions. It is also possible to add them dissolved in the same solvent when said reactants are soluble. It is also possible to add the reactants in paste or slurry form, in the same solvent.

The compound may be isolated by crystallisation from the same reaction solvent or by adding another solvent to cause precipitation and increase yield. Crystallisation, filtration and drying are preferred for reasons of quality and purity. Drying is effected preferably in an inert or low air atmosphere, such as at reduced pressure.

The end product is usually isolated hydrated and may be anhydrated by reduced pressure and heat or in the presence of a dessicant. In view of the relative hygroscopicity of the anhydrous product, it is preferable to isolate it as the monohydrate.

## Examples of application of the invention

To facilitate an understanding of the process of preparing the new compound of formula (I) of the invention, it is illustrated but not limited by the following examples:

### Example 1

A suspension of 8.80 g (40 mmoles) of L-5-HPT in 160 ml of a methanol-water mixture (1:1) under constant nitrogen flow, was heated to reflux. Once the L-5-HPT had dissolved, 1.55 g (19 mmoles) of zinc oxide (or 1.88 g of zinc hydroxide) in paste form in 40 ml of the same solvent was gradually added. Once the addition had terminated, while still hot, the reaction mixture was filtered and allowed to cool to room temperature. When an abundant precipitation had formed, the mass was cooled to 0 °C and held for one hour at that temperature. It was filtered, washed with methanol and allowed to dry for 1 hour at room temperature and 24 hours at reduced pressure in the presence of phosphorous pentoxide.

A white or ivory coloured crystalline solid having the following characteristics was obtained:
M.p.: decomposes over 300 °C without melting. IR (KBr) : 3600-2600 (broad band), 1614, 1492, 1459, 1388, 1346, 1236, 1200, 1111, 935 and 793 $cm^{-1}$
UV max. ($CH_3OH$, 0.1N NaOH, 0.1N HCl): 277 ± 2nm

| Elementary analysis for $C_{22}H_{22}N_4O_6Zn.H_2O$ (%): | |
|---|---|
| Calculated (%): | C50.64; H4.64; N10.74; Zn12.53 ($H_2O$: 3.45%) |
| Found (%) : | C50.39; H4.69; N10.55; Zn12.55 ($H_2O$: phosphorous pentoxide, 110 °C and reduced pressure 3.54%; K.F. 3.73%) |

Example 2

38 ml of 1N sodium hydroxide were added slowly over 8.80 g (40 mmoles) of L-5-HPT in 250 ml of a methanol-water mixture (1:1) under a constant nitrogen flow. Once the addition was terminated, the addition of a solution of $ZnCl_2$, 2.59 g (19 mmoles) in 50 ml of the same solvent was started. At the end of this addition, the reaction mass was raised to reflux and held there for 30 minutes. The mixture was allowed to cool to room temperature and held at that temperature for two hours. It was cooled to 0 °C and held there for 1 hour. It was filtered, washed with a methanol-water mixture (1:1) and methanol and allowed to dry for 30 minutes at room temperature in air, after which it was dried for 24 hours at reduced pressure in the presence of phosphorous pentoxide.

A crystalline solid having the same analytical characteristics as the previous example was thus obtained.

Therapeutical applications

The new formula (I) compound according to the invention is effective mainly on the central nervous system and has the following therapeutical applications:

Treatment of central nervous system disorders. Treatment of depression and of all forms of depressive states. Treatment of excitation and exhaustion states and of sleep disorders. Treatment of mental alterations and disorders and of maladjusted personality.

Pharmaceutical forms

For therapeutical use, the new L-5-hydroxytryptophane zinc compound of formula (I) of the present invention will normally be administered in pharmaceutical forms comprising as essential active ingredient at least the said compound, alone or associated with a pharmaceutical vehicle therefor.

The pharmaceutical vehicle used may be, for example, a solid or a liquid. Examples of solid vehicles : lactose, terra alba, sucrose, talc, gelatine, agar, pectin, acacia, magnesium stearate, stearic acid and the like. Examples of liquid vehicles are: syrup, groundnut oil, olive oil, water and the like.

Consequently, it will be appreciated that a wide range of pharmaceutical forms may be used. Thus, if a solid vehicle is used, the formula (I) compound may be prepared in pill, hard gelatine capsule, powder, granule, tablets, etc. form.

If a liquid vehicle is used, the preparation with the formula (I) active compound may adopt the form of syrup, emulsion, soft gelatine capsule, sterile liquid injectable solution as ampoule or aqueous or non-aqueous suspension, etc.

The pharmaceutical forms are prepared following conventional galenic techniques using processes such as mixing, granulating and pressing or dissolution of the ingredients, as appropriate for the desired preparation.

The active compound will be present in a pharmaceutical form in an effective amount varying between 100 mg and 1 g, depending on the conditions of each patient and the physician's judgement. The way of administration is unlimited with oral, parenteral, rectal, etc administration being possible.

Other pharmacologically active compounds may be included in the same pharmaceutical form.

**Claims**

1. A compound of formula (I):

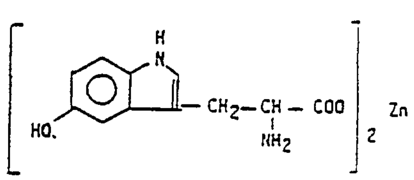

(I)

2. A process tor the preparation of a compound according to claim 1, **characterised** in that L-5-hydroxytryptophane of formula (II)

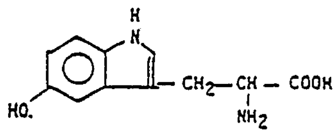

(II)

or an ammonium, alkali metal or alkaline earth metal salt thereof is reacted with zinc oxide, hydroxide or a zinc salt in a polar solvent.

3. A process according to claim 2, **characterised** in that said polar solvent is water or a methanol-water mixture.

4. A process according to claim 2 or 3, **characterised** in that the reaction temperature is equal to or lower than the reflux temperature of the solvent.

5. A process according to any one of the above claims 2 to 4, **characterised** in that said zinc derivative is isolated by crystallisation from the same solvent, followed by filtration and drying.

6. A pharmaceutical formulation comprising a compound of formula (I) together with a pharmaceutically acceptable carrier therefor.

7. A pharmaceutical formulation according to claim 6 for use in the treatment of central nervous system disorders.

8. A compound of formula (I) for use in the preparation of a medicament for the treatment of CNS disorders.